# FASCICULE DE BREVET EUROPEEN

(11) **EP 2 063 936 B1**
(45) Date de publication et mention de la délivrance du brevet: **23.11.2016**
(21) Numéro de dépôt: 07823462.2
(22) Date de dépôt: 31.08.2007
(51) Int. Cl.: A61M 5/31, A61M 5/28, A61M 5/20, A61M 5/30

(54) **DISPOSITIF D'INJECTION SANS AIGUILLE MUNI D'UN RESERVOIR SÉCURISÉ**
NADELLOSE INJEKTIONSVORRICHTUNG MIT GESCHÜTZTEM RESERVOIR
NEEDLELESS INJECTION DEVICE EQUIPPED WITH A PROTECTED RESERVOIR

(30) Priorité: 19.09.2006 FR 0608166
(43) Date de publication de la demande: 03.06.2009
(73) Titulaire: Crossject, 75004 Paris (FR)
(72) Inventeur: ALEXANDRE, Patrick, F-70100 Gray (FR)
(74) Mandataire: Gaillarde, Frédéric F. Ch.
(86) Numéro de dépôt international: PCT/FR2007/001417
(87) Numéro de publication internationale: WO 2008/034960

(56) Documents cités:
- WO-A-01/97884
- FR-A1- 2 853 836
- FR-A1- 2 853 837
- FR-A1- 2 865 407

## Description

Le domaine technique de l'invention est celui des dispositifs d'injection sans aiguille, pré-remplis et jetables, fonctionnant avec une source d'énergie comme, par exemple, un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection, ou être constitué d'un solide pulvérulent mis en suspension à une certaine concentration dans un liquide approprié. La granulométrie du principe actif doit alors être compatible avec le diamètre des conduits pour éviter de les obturer.

Les dispositifs d'injection sans aiguille comportant un réservoir de principe actif en forme de tube muni d'une collerette existent déjà et ont fait l'objet de brevets. On peut citer, par exemple, la demande de brevet FR 2 853 837 qui se rapporte à un dispositif d'injection sans aiguille comprenant une buse d'injection et un tube destiné à recevoir un principe actif à injecter, ledit tube venant se fixer sur ladite buse à l'aide de moyens de liaison. La principale caractéristique technique de ce dispositif d'injection est que le tube contenant le principe actif liquide comporte une collerette qui va venir s'emboîter dans des moyens de liaison fixés sur ladite buse pour solidariser de façon solide et fiable ledit tube à ladite buse. La collerette a une fonction purement mécanique qui est celle d'une butée de fixation. On peut citer également le document WO 01/97884 A qui décrit une seringue sans aiguille qui est apte à injecter une quantité variable de principe actif en adaptant, par déplacement d'un bouchon-piston aval dans le tube, le volume compris entre les deux bouchons-pistons à la quantité de principe actif à injecter.

On connaît également le document FR 2 865 407 A1 qui décrit une seringue sans aiguille comportant une buse striée visant à amortir l'impact de l'ensemble mobile quand l'obturateur aval arrive au contact du fond de l'alésage du réceptacle et aussi celui d'éviter les rebonds dudit obturateur aval après cet impact. On connaît également le document FR 2 853 836 A1, qui vise à résoudre les problèmes d'optimisation des entrées dans les conduits d'injection de la buse et ceux de réduction des efforts de pression sur le réceptacle pendant la phase d'injection.

Dans ce but, selon ce document FR 2 853 836 A1, chaque entrée dans les conduits d'injection comprend un lamage positionné raccordé à un canal radial débouchant dans l'alésage central.

Le but de l'invention est de minimiser voire d'éliminer les risques d'endommagement du tube contenant le principe actif liquide et du stoppeur aval dans la configuration spécifique du «double stoppeur». Ladite configuration se caractérise par la présence d'une colonne de liquide délimitée d'une part, par la paroi latérale du tube et, d'autre part, par un stoppeur amont et un stoppeur aval entre lesquels est logé le principe actif liquide. Sous l'effet de la génération de gaz, ladite colonne se déplace dans le tube jusqu'à ce que le stoppeur aval vienne au contact du fond d'un réceptacle situé dans la buse, de façon à libérer des canaux périphériques d'injection pour expulser ledit principe actif. Lorsque le stoppeur aval impacte le fond dudit réceptacle, il crée une onde de choc qui va se proroger jusqu'au tube et dont l'intensité est maximale à l'extrémité dudit tube qui est au contact de la buse. Pour résister à cette onde de choc et donc éviter au tube qu'il ne se brise, l'épaisseur dudit tube au niveau de ladite extrémité a été accrue par la combinaison d'une collerette et d'un rétrécissement interne dudit tube. Ledit rétrécissement permet également de précontraindre le stoppeur aval en le déformant afin de faciliter sa pénétration dans le réceptacle. Pour résumer, le rétrécissement du tube permet à la fois d'accroître l'épaisseur du tube afin d'augmenter sa résistance à une onde de choc, et de déformer le stoppeur aval afin de faciliter son passage dans le réceptacle. En effet, dans une configuration « double stoppeur », le stoppeur aval et le tube en verre sont les deux éléments les plus sollicités lors du fonctionnement du dispositif d'injection sans aiguille.

Dans la description et les revendications, le terme « longueur » correspond à une dimension prise suivant l'axe de rotation du tube et le terme « épaisseur » correspond à une dimension prise suivant un axe radial dudit tube. De plus, les termes « stoppeur » et « bouchon-piston » sont équivalents.

Plus spécifiquement, l'invention se rapporte à un dispositif d'injection sans aiguille comprenant un générateur de gaz, un réservoir constitué par un tube en verre obturé par un stoppeur amont et un stoppeur aval entre lesquels est logé un principe actif liquide, et une buse d'injection munie d'un réceptacle et d'au moins un conduit périphérique d'injection, ledit tube possédant à l'une de ses extrémités une collerette par l'intermédiaire de laquelle il est au contact de ladite buse. La principale caractéristique de ce dispositif est que le tube présente un canal interne comportant une partie amont prolongée par une partie aval de plus faible diamètre, ladite partie aval étant entourée par ladite collerette et débouchant dans le réceptacle.

De cette manière, l'extrémité du tube, qui est au contact de la buse et qui représente la zone dudit tube la plus sollicitée par l'onde de choc réfléchie par le fond du réceptacle suite à l'impact du stoppeur aval, possède une épaisseur accrue grâce, d'une part, à la présence de la collerette et, d'autre part, au rétrécissement de ladite partie aval.

Avantageusement, le stoppeur est en matériau déformable de sorte que, sous l'effet du gaz, il se déplace dans le rétrécissement du tube en se déformant, avant de pénétrer dans le réceptacle.

De façon préférentielle, la longueur de la partie aval du tube est supérieure ou égale à la longueur de la collerette.

De façon avantageuse, le canal interne présente une partie convergente séparant la partie amont de la partie aval. Ainsi, le passage du stoppeur aval de la partie amont du tube vers la partie aval de plus faible diamètre s'effectue progressivement en facilitant sa déformation, au contraire d'un épaulement droit qui présenterait une arête saillante susceptible d'endommager ledit stoppeur lors de son passage.

Préférentiellement, le rapport entre le diamètre de la partie aval et le diamètre de la partie amont est compris entre 0,85 et 0,98. De façon avantageuse, ledit rapport vaut 0,95.

Avantageusement, le rapport entre la longueur de la partie aval du canal interne et la longueur du stoppeur est compris entre 0,5 et 2,0, de préférence entre 0,6 et 1,2.

Avantageusement, le rapport entre l'épaisseur du tube au niveau de la partie amont et l'épaisseur du tube au niveau de la collerette est inférieur à 0,5.

Avantageusement, le rapport entre la longueur de la collerette et l'épaisseur du tube au niveau de ladite collerette est compris entre 0,1 et 0,8, de préférence entre 0,15 et 0,25).

De façon avantageuse, le stoppeur aval est plein et est de forme cylindrique présentant une paroi latérale externe lisse et présentant à chacune de ses extrémités une face circulaire plane. Autrement dit, le stoppeur présente globalement une forme lisse, sans aucune aspérité susceptible d'être arrachée ou découpée au moment du passage dudit stoppeur dans la partie rétrécie du tube.

Préférentiellement, le tube en verre est traité pour améliorer sa résistance par trempage chimique, par exemple à l'aide de bains de sels de potassium en fusion, ou par trempage thermique, les niveaux de précontraintes de compression à atteindre en surface étant au minimum de 100 MPa.

De façon avantageuse, le tube en verre et le stoppeur aval sont siliconés pour améliorer notamment les qualités de glissement du stoppeur aval dans la partie rétrécie du tube.

De façon préférentielle, le générateur de gaz est un générateur de gaz pyrotechnique possédant un système d'allumage et une charge pyrotechnique.

Les dispositifs d'injection sans aiguille selon l'invention présentent l'avantage de posséder un niveau de sécurité élevé, dans la mesure où les caractéristiques techniques desdites seringues permettent d'éviter la rupture du tube en verre et l'endommagement, voire la fragmentation, du stoppeur aval lors de son passage dans la partie rétrécie dudit tube. Lesdits dispositifs présentent également l'avantage d'être plus fiables en incorporant un mécanisme de fonctionnement simple fournissant une certaine fluidité de déplacement des pièces impliquées.

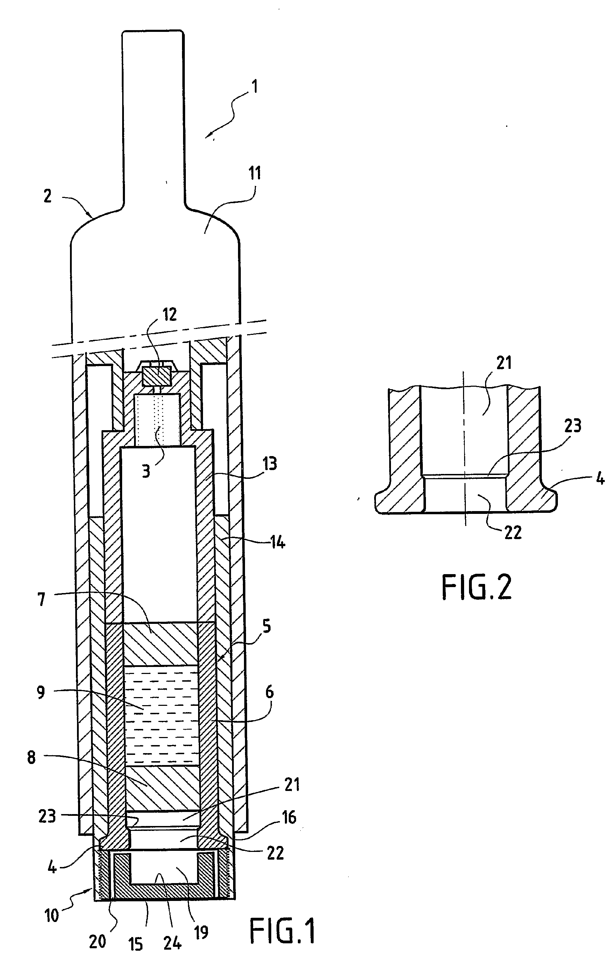

On donne ci-après la description détaillée de deux modes de réalisation préférés de l'invention en se référant aux figures 1 et 2.
- La figure 1 est une vue en coupe axiale longitudinale partielle d'un dispositif d'injection sans aiguille selon l'invention.
- La figure 2 est une vue en coupe axiale longitudinale de la partie rétrécie du tube d'un dispositif d'injection sans aiguille selon l'invention.

En se référant à la figure 1, un dispositif d'injection sans aiguille 1 selon l'invention comprend un générateur pyrotechnique 2 de gaz composé d'un système d'initiation et d'une charge pyrotechnique 3, un réservoir 5 constitué par un tube 6 en verre obturé par un bouchon-piston amont 7 et un bouchon-piston aval 8 entre lesquels est contenu le principe actif liquide 9, et un dispositif d'injection 10. Le tube 6 est avantageusement réalisé en un verre borosilicaté de type I.

Le système d'initiation fait intervenir un dispositif de percussion 11 non détaillé sur la figure 1 et une amorce 12. Le dispositif de percussion 11 qui est déclenché par un bouton-poussoir comprend un ressort et une masselotte munie d'un percuteur (non représentés). La masselotte est bloquée par au moins une bille coincée entre ladite masselotte et le bouton-poussoir, et ledit bouton-poussoir possède une gorge interne circulaire.

La charge pyrotechnique 3 débouche dans un corps creux 13 sensiblement cylindrique, lui-même prolongé par le tube 6 du réservoir 5, ledit tube 6 ayant le même diamètre interne que celui du corps creux 13. Le tube 6 est en continuité du corps creux 13 en étant à son contact, ces deux pièces 6, 13 ayant également le même diamètre externe. Celles-ci sont donc parfaitement alignées l'une sur l'autre et sont maintenues dans cette configuration par une enveloppe 14 en matière plastique exerçant, après montage, une légère compression sur le corps creux 13 ainsi que sur le tube 6. L'enveloppe 14 prend naissance environ à mi-longueur du corps creux 13 et se prolonge au-delà du tube 6 en verre par une avancée cylindrique creuse dont la paroi latérale interne est filetée. En se référant à la figure 2, le tube 6 présente donc une extrémité amont qui est au contact du corps creux 13 et une extrémité aval munie d'une collerette 4 annulaire, entourant ladite extrémité et se retrouvant en position distale par rapport au centre dudit tube 6. Le tube 6 présente un canal interne cylindrique comportant une partie amont 21 prolongée par une partie aval 22 de plus faible diamètre, ladite partie aval étant entourée par ladite collerette 4. La partie amont 21 débouche dans la partie aval 22 par l'intermédiaire d'une partie convergente 23 dont le diamètre le plus élevé correspond au diamètre de ladite partie amont 21 et le diamètre le plus faible correspond au diamètre de ladite partie aval 22. L'extrémité du tube 6 munie de la collerette 4 présente une surface annulaire plane.

Une pièce cylindrique creuse 15 jouant le rôle d'une buse d'injection, et présentant sur sa surface latérale externe un filetage, est vissée dans l'avancée cylindrique creuse filetée de l'enveloppe 14. La pièce creuse 15 possède quatre conduits périphériques d'injection 20, parallèles entre eux et traversant ladite pièce creuse 15 sur toute sa longueur. Lesdits conduits 20 sont uniformément répartis autour de la pièce creuse 15. Ladite pièce creuse 15 comporte un réceptacle cylindrique sous la forme d'un évidement central présentant un fond plat 24.

Lorsque la pièce creuse 15 est vissée dans l'avancée de l'enveloppe 14, chaque conduit 20 longitudinal se trouve prolongé par un canal radial débouchant sur la partie supérieure du réceptacle 19, ledit canal radial étant délimité à la fois par ladite pièce creuse 15 et l'extrémité du tube en verre 6 munie de la collerette 4. De façon plus précise, la pièce creuse 15 est vissée dans l'avancée jusqu'à ce que sa partie périphérique externe vienne en butée contre l'extrémité du tube 6 munie de la collerette 4. Le réceptacle 19 étant délimité par une partie annulaire interne dont la longueur est plus faible que celle de la partie périphérique externe, lesdites parties interne et externe étant séparées l'une de l'autre par les conduits d'injection 20, un espace est ménagé entre ladite partie annulaire interne et l'extrémité du tube, en prolongation de chacun des conduits périphériques 20 traversant la buse 15.

Le mode de fonctionnement de ce mode de réalisation préféré de l'invention s'effectue comme suit. L'utilisateur positionne le dispositif d'injection sans aiguille 1 de façon à ce que son extrémité vienne en appui contre la peau du patient à traiter. Une pression sur le bouton poussoir permet de le faire coulisser le long du dispositif d'injection sans aiguille 1 jusqu'à ce que la gorge vienne au niveau de la bille qui bloque la masselotte. La bille en se dégageant dans la gorge libère la masselotte qui, sous l'effet du ressort qui se détend, est propulsée vers l'amorce 12, le percuteur en avant. L'amorce 12 qui est alors initiée provoque la mise à feu de la charge pyrotechnique 3. Les gaz émis vont exercer une pression sur le bouchon-piston amont 7 et la colonne de liquide constituée par les bouchons-pistons amont 7 et aval 8 et le principe actif liquide 9 va amorcer un déplacement dans le réservoir 5. Le bouchon-piston aval 8 va alors traverser la partie aval rétrécie du tube 6 en se déformant sans se découper ou se fragmenter grâce à la partie convergente 23 dudit tube 6, avant de venir se loger dans le réceptacle 19. Sous l'effet de la pression, ledit bouchon-piston aval 8 se déforme en « s'écrasant » au fond du réceptacle 19 permettant alors de mettre en contact le principe actif liquide 9 et les quatre conduits périphériques d'injection 20 de la buse 15. Ledit liquide 9 est alors expulsé vers la peau du patient à traiter et cette injection dure jusqu'à ce que le bouchon-piston amont 7 vienne au contact du bouchon piston aval 8 dans le réceptacle 19.

## Revendications

1. Dispositif d'injection sans aiguille (1) comprenant un générateur de gaz (2), un réservoir (5) constitué par un tube (6) en verre obturé par un stoppeur amont (7) et un stoppeur aval (8) entre lesquels est logé un principe actif liquide (9), et une buse d'injection (15) munie d'un réceptacle (19) et d'au moins un conduit périphérique d'injection (20), ledit tube (6) possédant à l'une de ses extrémités une collerette (4) par l'intermédiaire de laquelle il est au contact de ladite buse (15), le tube (6) présentant un canal interne comportant une partie amont (21) ayant le même diamètre interne que celui du réservoir (5) et étant prolongée par une partie aval (22), ladite partie aval (22) étant entourée par ladite collerette (4) et débouchant dans le réceptacle (19), **caractérisé en ce que** la partie aval (22) est de plus faible diamètre que la partie amont (21).

2. Dispositif d'injection sans aiguille (1) selon la revendication 1, **caractérisé en ce que** le canal interne présente une partie convergente (23) séparant la partie amont (21) de la partie aval (22).

3. Dispositif d'injection sans aiguille (1) selon l'une quelconque des revendications 1 ou 2, **caractérisé en ce que** le rapport entre le diamètre de la partie aval (22) et le diamètre de la partie amont (21) est compris entre 0,85 et 0,98.

4. Dispositif d'injection sans aiguille (1) selon la revendication 1, **caractérisé en ce que** le rapport entre la longueur de la partie aval (22) du canal interne et la longueur du stoppeur aval (8) est compris entre 0,5 et 2,0.

5. Dispositif d'injection sans aiguille (1) selon la revendication 1, **caractérisé en ce que** la longueur de la partie aval (22) est supérieure ou égale à la longueur de la collerette (4).

6. Dispositif d'injection sans aiguille (1) selon la revendication 5, **caractérisé en ce que** le rapport entre l'épaisseur du tube (6) au niveau de la partie amont (21) et l'épaisseur du tube au niveau de la collerette (4) est inférieur à 0,5.

7. Dispositif d'injection sans aiguille (1) selon la revendication 5, **caractérisé en ce que** le rapport entre la longueur de la collerette (4) et l'épaisseur du tube (16) au niveau de ladite collerette (4) est compris entre 0,1 et 0,8.

8. Dispositif d'injection sans aiguille (1) selon la revendication 1, **caractérisé en ce que** le stoppeur aval (8) est plein et est de forme cylindrique présentant une paroi latérale externe lisse et présentant à chacune de ses extrémités une face circulaire plane.

9. Dispositif d'injection sans aiguille (1) selon la revendication 1, **caractérisé en ce que** le tube (6) en verre est traité pour améliorer sa résistance par trempage chimique ou par trempage thermique.

10. Dispositif d'injection sans aiguille (1) selon la revendication 1, **caractérisé en ce que** le tube (6) en verre et le stoppeur aval (8) sont siliconés.

## Patentansprüche

1. Nadellose Injektionsvorrichtung (1), einen Gasgenerator (2) umfassend, ein Reservoir (5), das durch eine Röhre (6) aus Glas gebildet wird, die durch einen vorgelagerten Stopper (7) und einen nachgelagerten Stopper (8) verschlossen wird, zwischen denen ein flüssiger Wirkstoff (9) untergebracht ist, und eine Injektionsdüse (15), die mit einem Aufnahmebehälter (19) und mit zumindest einer peripheren Injektionsleitung (20) versehen ist, wobei die besagte Röhre (6) an einem ihrer Enden einen Bund (4) besitzt, über den sie mit der besagten Düse (15) in Kontakt steht, wobei die Röhre (6) einen internen Kanal aufweist, der einen vorgelagerten Abschnitt (21) umfasst, der denselben Innendurchmesser aufweist, wie jener des Reservoirs (5) und sich in einem nachgelagerten Abschnitt (22) fortsetzt, wobei der besagte nachgelagerte Abschnitt (22) vom besagten Bund (4) umgeben ist, und in den Aufnahmebehälter (19) mündet, **dadurch gekennzeichnet, dass** der nachgelagerte Abschnitt (22) einen kleineren Durchmesser aufweist, als der vorgelagerte Abschnitt (21).

2. Nadellose Injektionsvorrichtung (1) nach Anspruch 1, **dadurch gekennzeichnet, dass** der interne Kanal einen konvergierenden Abschnitt (23) aufweist, der den vorgelagerten Abschnitt (21) vom nachgelagerten Abschnitt (22) trennt.

3. Nadellose Injektionsvorrichtung (1) nach einem der Ansprüche 1 oder 2, **dadurch gekennzeichnet, dass** das Verhältnis zwischen dem Durchmesser des nachgelagerten Abschnitts (22) und dem Durchmesser des vorgelagerten Abschnitts (21) zwischen 0,85 und 0,98 liegt.

4. Nadellose Injektionsvorrichtung (1) nach Anspruch 1, **dadurch gekennzeichnet, dass** das Verhältnis zwischen der Länge des nachgelagerten Abschnitts (22) des internen Kanals und der Länge des nachgelagerten Stoppers (8) zwischen 0,5 und 2,0 liegt.

5. Nadellose Injektionsvorrichtung (1) nach Anspruch 1, **dadurch gekennzeichnet, dass** die Länge des nachgelagerten Abschnitts (22) größer oder gleich der Länge des Bundes (4) ist.

6. Nadellose Injektionsvorrichtung (1) nach Anspruch 5, **dadurch gekennzeichnet, dass** das Verhältnis zwischen der Stärke der Röhre (6) im Bereich des vorgelagerten Abschnitts (21) und der Stärke der Röhre im Bereich des Bundes (4) kleiner als 0,5 ist.

7. Nadellose Injektionsvorrichtung (1) nach Anspruch 5, **dadurch gekennzeichnet, dass** das Verhältnis zwischen der Länge des Bundes (4) und der Stärke der Röhre (16) im Bereich des besagten Bundes (4) zwischen 0,1 und 0,8 liegt.

8. Nadellose Injektionsvorrichtung (1) nach Anspruch 1, **dadurch gekennzeichnet, dass** der nachgelagerte Stopper (8) voll ist und eine zylindrische Form aufweist, die eine glatte äußere Seitenwand aufweist und an jedem seiner Enden eine flache kreisförmige Fläche aufweist.

9. Nadellose Injektionsvorrichtung (1) nach Anspruch 1, **dadurch gekennzeichnet, dass** die Röhre (6) aus Glas behandelt ist, um ihre Beständigkeit durch chemisches Härten oder durch thermisches Härten zu verbessern.

10. Nadellose Injektionsvorrichtung (1) nach Anspruch 1, **dadurch gekennzeichnet, dass** die Röhre (6) aus Glas und der nachgelagerte Stopper (8) silikonhaltig sind.

## Claims

1. A needleless injection device (1) comprising a gas generator (2), a reservoir (5) consisting of a glass tube (6) closed by an upstream stopper (7) and a downstream stopper (8) between which a liquid active principle (9) is housed, and an injection nozzle (15) provided with a receptacle (19) and at least one peripheral injection conduit (20), said tube (6) having, at one of its ends, a collar (4) through which it is in contact with said nozzle (15), the tube (6) having an inner channel including an upstream portion (21) having the same inner diameter as that of the reservoir (5) and being extended by a downstream portion (22), said downstream portion (22) being surrounded by said collar (4) and opening into the receptacle (19), **characterized in that** the upstream portion (22) has a diameter smaller than the upstream portion (21).

2. The needleless injection device (1) according to claim 1, **characterized in that** the inner channel has a convergent portion (23) separating the upstream portion (21) from the downstream portion (22).

3. The needleless injection device (1) according to any one of claims 1 or 2, **characterized in that** the ratio between the diameter of the downstream portion (22) and the diameter of the upstream portion (21) is comprised between 0.85 and 0.98.

4. The needleless injection device (1) according to claim 1, **characterized in that** the ratio between the length of the downstream portion (22) of the inner channel and the length of the downstream stopper (8) is comprised between 0.5 and 2.0.

5. The needleless injection device (1) according to claim 1, **characterized in that** the length of the downstream portion (22) is greater than or equal to the length of the collar (4).

6. The needleless injection device (1) according to claim 5, **characterized in that** the ratio between the thickness of the tube (6) at the upstream portion (21) and the thickness of the tube at the collar (4) is lower than 0.5.

7. The needleless injection device (1) according to claim 5, **characterized in that** the ratio between the length of the collar (4) and the thickness of the tube (16) at said collar (4) is comprised between 0.1 and 0.8.

8. The needleless injection device (1) according to claim 1, **characterized in that** the downstream stopper (8) is solid and has a cylindrical shape with a smooth outer lateral wall and, at each of its ends, a flat circular face.

9. The needleless injection device (1) according to claim 1, **characterized in that** the glass tube (6) is treated to improve its resistance by chemical dipping or by heat dipping.

10. The needleless injection device (1) according to claim 1, **characterized in that** the glass tube (6) and the downstream stopper (8) are siliconized.
